# EUROPÄISCHE PATENTSCHRIFT

(11) **EP 1 851 322 B1**
(45) Veröffentlichungstag und Bekanntmachung des Hinweises auf die Patenterteilung: **21.10.2009**
(21) Anmeldenummer: 06723073.0
(22) Anmeldetag: 21.02.2006
(51) Int. Cl.: C12P 7/02

(54) **VERFAHREN ZUR ENANTIOSELEKTIVEN ENZYMATISCHEN REDUKTION VON KETOVERBINDUNGEN**
METHOD FOR THE ENANTIOSELECTIVE ENZYMATIC REDUCTION OF KETO COMPOUNDS
PROCEDE DE REDUCTION ENZYMATIQUE ENANTIOSELECTIVE DE COMPOSES CETONIQUES

(30) Priorität: 21.02.2005 AT 2852005
(43) Veröffentlichungstag der Anmeldung: 07.11.2007
(73) Patentinhaber: IEP GmbH, 65203 Wiesbaden (DE)
(72) Erfinder: GUPTA, Antje, 65207 Wiesbaden (DE); BOBKOVA, Maria, 65510 Idstein (DE); TSCHENTSCHER, Anke, 65347 Eltville-Hattenheim (DE)
(74) Vertreter: Schwarz, Albin
(86) Internationale Anmeldenummer: PCT/EP2006/001562
(87) Internationale Veröffentlichungsnummer: WO 2006/087235

(56) Entgegenhaltungen:
- EP-A- 0 796 914
- EP-A- 1 179 595
- WO-A-02/086126
- WO-A-03/078615
- WO-A-20/04111083
- YANG H ET AL: "The enantiomeric purity of alcohols formed by enzymatic reduction of ketones can be improved by optimisation of the temperature and by using a high co-substrate concentration" BBA - GENERAL SUBJECTS, ELSEVIER SCIENCE PUBLISHERS, NL, Bd. 1336, Nr. 1, 19. Juli 1997 (1997-07-19), Seiten 51-58, XP004276020 ISSN: 0304-4165

## Beschreibung

Die vorliegende Erfindung betrifft ein Verfahren zur enantioselektiven enzymatischen Reduktion von Ketoverbindungen, insbesondere von 4-Halo-3-oxobuttersäureestern zu den entsprechenden R-Alkoholen bzw. S-4-Halo-3-hydroxybuttersäureestern.

Carbonylreduktasen (weitere Bezeichnungen: Alkoholdehydrogenasen, Oxidoreduktasen) sind als Katalysatoren zur Reduktion von Carbonylverbindungen bzw. zur Oxidation von sekundären Alkoholen bekannt. Diese Enzyme benötigen ein Co-Enzym, z.B. NAD(P)H. Die Reduktion von Ketonen mit der aus Lactobacillus kefir gewonnenen Carbonylreduktase und dem Co-Enzym NADPH ist z.B. aus der US 5,342,767 bekannt.

Optisch aktive Hydroxyverbindungen sind wertvolle chirale Bausteine mit breiter Anwendung für die Synthese von pharmakologisch wirksamen Verbindungen, aromatischen Substanzen, Pheromonen, Agrochemikalien und Enzyminhibitoren. S-4-Halo-3-hydroxybuttersäureester beispielsweise sind wichtige Intermediate für die Synthese von HMG-CoA Reduktase Inhibitoren, D-Carnitin und anderen.

Es sind enantioselektive Enzyme bekannt, die beispielsweise in der Lage sind, 4-Halo-3-oxobuttersäureester zu den entsprechenden S-4-halo-3-hydroxybutter-säureestern zu reduzieren. Als Beispiele sind zu nennen:
Reduktasen aus Bäckerhefe (D-enzyme-1, D-enzyme-2, J. Am. Chem. Soc. 107, 2993-2994, 1985);
Aldehydreduktase 2 aus Sporobolomyces salmonicolor (Appl. Environ. Microbiol. 65, 5207-5211, 1999);
Ketopantothensäureester Reduktase aus Candida macedoniensis (Arch. Biochem. Biophys. 294, 469-474, 1992);
Reduktase aus Geotrichum candidum (Enzyme Mircrob. Technol. 14, 731-738, 1992); Carbonylreduktase aus Candida magnoliae (WO 98/35025);
Carbonyreduktase aus Kluyveromyces lactis (JP-A Hei 11-187869);
β-Ketoacyl-acyl carrier protein Reduktase der Fettsäuresynthetase Typ II (JP-A 2000-189170);
(R)-2-Oktanol Dehydrogenase aus Pichia finlandica (EP 1179595 A1);
R-spezifische sekundären Alkoholdehydrogenasen aus Organismen der Gattung *Lactobacillus* (Lactobacillus kefir (US5200335), Lactobacillus brevis (DE 19610984 A1) (Acta Crystallogr D Biol Crystallogr. 2000 Dec;56 Pt 12:1696-8), Lactobacillus minor (DE10119274) ;
*Pseudomonas* (US 05385833)(Appl Microbiol Biotechnol. 2002 Aug;59(4-5):483-7. Epub 2002 Jun 26.,J. Org. Chem. 1992, 57, 1532).

Mit Ausnahme der Enzyme aus Pseudomonas, aus Lactobacillus und aus Pichia finlandica (EP 1179595 A1) akzeptieren die bekannten Enzyme meist keine sekundären Alkohole als Substrate und katalysieren auch nicht die Oxidation sekundärer Alkohole.

In einem industriellen enzymatischen Reduktionsprozess müssen diese Enzyme daher an ein weiteres, für die Regeneration des Cofaktors NADH bzw. NADPH verantwortliches Enzym gekoppelt werden. Solche für die Regeneration von NAD(P)H geeigneten Enzyme sind Formiatdehydrogenase, Glucosedehydrogenase, Malatdehydrogenase, Glyceroldehydrogenase und Alkoholdehydrogenase, welche bevorzugt zusammen mit dem Enzym zur Reduktion von 4-Halo-3-oxobuttersäureestern exprimiert werden.

Es konnte gezeigt werden, dass rekombinante Zellen von Escherichia coli, die beispielsweise gleichzeitig das Gen für die Carbonylreduktase aus Candida magnoliae, sowie das Gen für die Glucosedehydrogenase aus Bacillus megaterium exprimieren, effizient im wässrig/organischen Zwei-Phasensystem eingesetzt werden können, wobei Substratkonzentrationen von > 40 % (Gewichtsprozent) realisiert wurden (Appl Microbiol Biotechnol (2001), 55; 590-595, AnnN Y Acad Sci. 1998 Dec 13;864:87-95).

Prozesse mit Enzymen aus der Gruppe der Lactobacillales (Lactobacillus minor; DE 10119274) wurden bisher erfolgreich mit substratgekoppelter Coenzymregenerierung mit 2-Propanol realisiert, wobei die Reduktion unlöslicher Substrate auch in hohen Konzentrationen durch Einsatz wässrig/organischer Zwei-Phasensysteme realisiert wurde (US 5342767, DE10119274).

Grundsätzlich wurde bei der Anwendung der Substrat-gekoppelten Coenzymregenerierung mit 2-Propanol bzw. 2-Butanol die geringe Toleranz der meisten Enzyme gegenüber 2-Propanol und 2-Butanol als limitierend angesehen. Üblicherweise werden Konzentrationen an 2-Propanol von deutlich unter 10 Vol.-% eingesetzt.

Es sind im Stand der Technik keine Verfahren bekannt, die die Verwendung von R-spezifischen Oxidoreduktasen aus Hefen mit substratgekoppelter Coenzymregenerierung mit 2-Propanol und/oder 2-Butanol beschreiben.

Durch den limitierten Einsatz des Cosubstrates 2-Propanol wurden nur unbefriedigende Substratkonzentrationen und Umsatzraten erreicht (Angew Chemie Int Ed Engl 2002, 41: 634-637, Biotechnol Bioeng 2004 Apr 5; 86 (1): 55-62).

In der EP 1 179 595 A wird ein Verfahren zur enantioselektiven Reduktion von Carbonylverbindungen beschrieben, bei dem die Carbonylverbindung in einer Konzentration von 0,1-90% und das Cosubstrat Glucose, Formiat, Ethanol oder 2-Propanol in 1-20 ' fachemÜberschuss eingesetzt werden.

Die WO 021086126 A lehrt ein Verfahren zur enantioselektiven Reduktion von Carbonylverbindungen, bei dem die S- oder R-spezifische Oxidoreduktase, der Co-Faktor NAD(P)H und die Carbonylverbindung in einem 2-Phasen-System inkubiert werden.

Die WO 20041111083 A und die WO 031078615 A beschreiben Verfahren zur Herstellung von Hydroxyverbindungen, wobei Carbonylverbindungen mit NADH als Co-Faktor und 2-Propanol oder 2-Butanol als Cosubstrat mittels einer S-spezifischen Oxidoreduktase aus Pichia capsulata bzw. Rhodococcus zu den entsprechenden S-Hydroxyverbindunegn reduziert werden.

Jüngst konnte eine S-spezifische, mittelkettige Alkoholdehydrogenase aus Rhodococcus ruber isoliert werden, die auch bei wesentlich höheren Konzentrationen von 2-Propanol 50-80 % (Volumsprozent) noch stabil und aktiv ist. (Biotechnol Bioeng 2004 Apr 5; 86 (1): 55-62), WO 03/078615).

Die Erfindung bezweckt die Überwindung dieser Nachteile und betrifft ein Verfahren zur enantioselektiven enzymatischen Reduktion von Ketoverbindungen der allgemeinen Formel I

R₁-C(O)-R₂ (I)

in der R1 für einen der Reste
1) -(C₁-C₂₀)-Alkyl, worin Alkyl geradkettig oder verzweigtkettig ist,
2) -(C₂-C₂₀)-Alkenyl, worin Alkenyl geradkettig oder verzweigtkettig ist und gegebenenfalls bis zu vier Doppelbindungen enthält,
3) -(C₂-C₂₀)-Alkinyl, worin Alkinyl geradkettig oder verzweigtkettig ist und gegebenenfalls bis zu vier Dreifachbindungen enthält,
4) -(C₆-C₁₄)-Aryl,
5) -(C₁-C₈)-Alkyl-(C₆₋C₁₄)-Aryl,
6) -(C₅-C₁₄)-Heterocyclus, der unsubstituiert oder ein-, zwei- oder dreifach durch -OH; Halogen, NO₂ und/oder -NH₂ substituiert ist, oder
7) -(C₃-C₇)-Cycloalkyl,
   steht, wobei die oben unter 1) bis 7) genannten Reste unsubstituiert sind oder unabhängig voneinander ein-, zwei- oder dreifach durch -OH, Halogen, NO₂ und/oder -NH₂ substituiert sind,
   und R₂ für einen der Reste
8) -(C₁-C₆)-Alkyl, worin Alkyl geradkettig oder verzweigtkettig ist,
9) -(C₂-C₆)-Alkenyl, worin Alkenyl geradkettig oder verzweigtkettig ist und gegebenenfalls bis zu drei Doppelbindwigen enthält,
10) -(C₂-C₆)-Alkinyl, worin Alkinyl geradkettig oder verzweigtkettig ist und gegebenenfalls zwei Dreifachbindungen enthält, oder
11) -(C₁-C₁₀)-Alkyl-C(O)-O-(C₁-C₆)-Alkyl, worin Alkyl gerade oder verzweigtkettig ist und unsubstituiert ist oder ein-, zwei- oder dreifach durch -OH, Halogen, -NO₂ und/oder -NH₂ substituiert ist,
   steht, wobei die oben unter 8) bis 11) genannten Reste unsubstituiert sind oder unabhängig voneinander ein-, zwei- oder dreifach durch -OH, Halogen, -NO₂ und/oder
   -NH₂ substituiert sind,
das dadurch gekennzeichnet ist, daß
eine flüssige, einphasige Mischung, welche
(a) mindestens 5 Gew./Vol.-% eine Verbindung der Formel (I),
(b) mindestens 15 Vol.-% 2-Propanol und/oder 2-Butanol, und
(c) Wasser
enthält, mit einer R-spezifischen Oxidoreduh-tase in Gegenwart eines Co-Faktors behandelt wird, um eine chirale Hydroxyverbindung der allgemeinen Formel II

R₁-CH(OH)-R₂ (II)

zu bilden, in welcher R₁ und R₂ die oben angegebene Bedeutung haben, wobei der Co-Faktor durch Oxidation des 2-Propanols und/oder 2-Butanols regeneriert wird.

Unter dem Begriff "Aryl" werden aromatische Kohlenstoffreste verstanden mit 6 bis 14 Kohlenstoffatomen im Ring. -(C₆-C₁₄)-Arylreste sind beispielsweise Phenyl, Naphthyl, zum Beispiel 1-Naphthyl, 2-Naphthyl, Biphenylyl, zum Beispiel 2-Biphenylyl, 3-Biphenylyl und 4-Biphenylyl, Anthryl oder Fluorenyl. Biphenylylreste, Naphthylreste und insbesondere Phenylreste sind bevorzugte Arylreste. Unter dem Begriff "Halogen" wird ein Element aus der Reihe Fluor, Chlor, Brom oder Jod verstanden. Unter demBegriff (C₁-C₂₀)-Alkyl" wird ein Kohlenwasserstoffrest verstanden, dessen Kohlenstoffkette geradkettig oder verzweigt ist und 1 bis 20 Kohlenstoffatome enthält beispielsweise Methyl, Ethyl, Propyl, Isopropyl, Butyl, tertiär-Butyl, Pentyl, Hexyl, Heptyl, Octyl, Nonenyl oder Decanyl. Unter dem Begriff "C₀-Alkyl" wird eine kovalente Bindung verstanden.

Unter dem Begriff "-(C₃-C₇)-cycloakyl" werden cyclische Kohlenwasserstoffreste verstanden, wie Cyclopropyl, Cylobutyl, Cyclopentyl, Cyclohexyl oder Cycloheptyl.

Der Begriff "-(C₅-C₁₄)-Heterocyclus" steht für einen monocyclischen oder bicyclischen 5-gliedrigen bis 14-gliedrigen heterocyclischen Ring, der teilweise gesättigt oder vollständig gesättigt ist. Beispiele für Heteroatome sind N, O und S. Beispiele für die Begriffe "-(C₅-C₁₄)-Heterocyclus" sind Reste, die sich von Pyrrol, Furan, Thiophen, Imidazol, Pyrazol, Oxazol, Isoxazol, Thiazol, Isothiazol, Tetrazol, 1,2,3,5-Oxathiadiazol-2-Oxide, Triazolone, Oxadiazolone, Isoxazolone, Oxadiazolidindione, Triazole, welche durch F, -CN, -CF₃ oder - C(O)-O-(C₁-C₄)-Alkyl substituert sind, 3-Hydroxypyrro-2,4-dione, 5-Oxo-1,2,4-Thiadiazole, Pyridin, Pyrazin, Pyrimidin, Indol, Isoindol, Indazol, Phthalazin, Chinolin, Isochinolin, Chinoxalin, Chinazolin, Cinnolin, -Carbolin und benz-anellierte, cyclopenta-, cyclohexa- oder cyclohepta-anellierten Derivaten dieser Heterocyclen ableiten. Insbesondere bevorzugt sind die Reste 2- oder 3-Pyrrolyl, Phenylpyrrolyl, wie 4- oder 5-Phenyl-2-pytxolyl, 2-Furyl, 2-Thienyl, 4-Imidazolyl, Methyl-imidazolyl, zum Beispiel 1-Methyl-2-, -4- oder -5-imidazolyl, 1,3-Thiazol-2-yl, 2-Pyridyl, 3-Pyridyl, 4-Pyridyl, 2-, 3- oder 4-Pyridyl-N-oxid, 2-Pyrazinyl, 2-, 4- oder 5-Pyrimidinyl, 2-, 3- oder 5-Indolyl, substituiertes 2-Indolyl, zum Beispiel 1-Methyl-, 5-Methyl-, 5-Methoxy-, 5-Benzyloxy-, 5-Chlor- oder 4,5-Dimethyl-2-indolyl, 1-Benzyl-2-oder -3-indolyl, 4,5,6,7-Teträhydro-2-indolyl, Cyclohepta[b]-5-pyrrolyl, 2-, 3- oder 4-Chinolyl, 1-, 3- oder 4-Isochinolyl, 1-Oxo-1,2-dihydro-3-isochinolyl, 2-Chinoxalinyl, 2-Benzofuranyl, 2-Benzo-thienyl, 2-Benzoxazolyl oder Benzothiazolyl oder Dihydropyridinyl, Pyrrolidinyl, zum Beispiel 2- oder 3-(N-Methylpyrrolidinyl), Piperazinyl, Morpholinyl, Thiomorpholinyl, Tetrahydrothienyl oder Benzodioxolanyl.

Die Erfindung betrifft auch analoge Verfahren zur enantioselektiven enzymatischen Reduktion der Ketoverbindung 2,5- Hexandion und der Ketoverbindung Acetoxyaceton.

Die vorliegende Erfindung beruht auf der Erkenntnis, daß bei Anwendung von R-spezifischen Alkoholdehydrogenasen bzw. Oxidoreduktasen diese auch bei Konzentrationen an 2-Propanol- und/oder 2-Butanol von weit über 15 Vol.-% und insbesondere über-25 % Vol.% eingesetzt werden können.

Dadurch eröffnet sich die Möglichkeit, auch gering wasserlösliche Substrate, wie beispielsweise 4-Halo-3-oxobuttersäureester in hoher Konzentration enzymatisch in einem homogenen, wässriglorganischem System enantioselektiv zu reduzieren. Dies ist insbesondere dann von Vorteil, wenn der entstehende chirale Alkohol in einem kontinuierlichen Prozess direkt, ohne vorherige Isolation einer in einem einphasigen, wässrig/organischen Reaktionsgemisch stattfindenden Folgereaktion zugeführt werden soll.

Dies ist beispielsweise bei der enantioselektiven Reduktion von 4-Chloracetoacetat der Fall, wo das entstehende Produkt S-4-Chlor-3-hydroxybuttersäureethylester in einem solchen homogenen Reaktionsgemisch direkt in ein Cyanisierungsverfahren eingespeist und zum (R) -4-cyano-3-hydroxybuttersäureethylester weiterverarbeitet werden kann (WO 03/097581 A1);

Unter dem Begriff "R-spezifische Oxidoreduktase" bzw. Alkoholdehydrogenase werden solche verstanden, die unsubstituierte Carbonylverbindungen, wie beispielsweise 2-Butanon, 2-Oktanon oder Acetophenon bevorzugt zu den entsprechenden R-Hydroxyverbindungen, wie beispielsweise R-2-Butanol, R-2-Oktanol oder R-2-Phenylethanol, reduzieren.

Die erfindungsgemäß eingesetzte R-speziEsche Oxidoreduktase ist vorzugsweise mikrobiellen Ursprungs und stammt insbesondere aus Bakterien der Gruppe Lactobacillales, insbesondere der Gattung *Lactobacillus,* z.B. Lactobacillus kefir (US 5,200,335), Lactobacillus brevis (DE 19610984 A1) (Acta Crystallogr D Biol Crystallogr. 2000 Dec;56 Pt 12:169b-8), Lactobacillus minor (DE10119274) oder *Leuconostoc carnosum,* oder aus Hefen, insbesondere der Gattungen *Pichia, Candida, Pachysolen, Debaromyces* oder *Issatschenkia,* besonders bevorzugt aus Pichia finlandica (EP 1 179 595 A1).

Im erfindungsgemäßen Verfahren wird als Co-Faktor bevorzugt NAD(p)H eingesetzt. Unter dem Begriff "NADPH" wird reduziertes Nicotinamid-adenin-dinucleotid-phosphat verstanden. Unter dem Begriff "NADI'" wird Nicotinamid-adenin-dinucleotid-phosphat verstanden.

Eine Ausführungsform des erfindungsgemäßen Verfahrens ist dadurch gekennzeichnet, däß die flüssige, einphasige Mischung bei Anwendung einer Oxidoreduktase bakteriellen Ursprungs mindestens 25 Vol.-% 2-Propanol und/oder 2-Butanol enthält.

Eine weitere Ausführungsform des erfindungsgemäßen Verfahrens besteht darin, daß die flüssige, einphasige Mischung zwischen 25 und 90 Vol.-%, insbesondere zwischen 35 und 70 Vol.-%, 2-Propanol und/oder 2-Butanol enthält.

In der flüssigen, einphasigen Mischung ist die Verbindung der allgemeinen Formel (I) bevorzugt zwischen 5 und 50 Gew./Vol.-%, insbesondere zwischen 15 und 50 Gew./Vol.%, enthalten.

Bei Anwendung einer Oxidoreduktase aus Hefen enthält die flüssige, einphasige Mischung bevorzugt mindestens 15 Vol.-% 2-Propanol.

Im erfindungsgemäßen Verfahren wird als Verbindung der allgemeinen Formel (I) bevorzugt Ethyl-4-chloracetoacetat, Methylacetoacetat, Ethyl-3-oxovaleriat, 4-Hydroxy-2-butanon, Ethylpyruvat, 2,3-Dichloracetophenon, 1-[3,5-Bis(trifluoromethyl)phenyl]ethan-1-one, Acetophenon, 2-Octanon, 3-Octanon, 1,4-Dichlor-2-butanon, Phenacylchlorid, Ethyl-4-bromoacetoacetat, 1,1-Dichloracetan, 1,1,3-Trichloraceton oder 1-Chlora,ceton eingesetzt.

Im erfindungsgemäßen Verfahren kann das Enzym entweder vollständig gereinigt, teilweise gereinigt oder in Zellen enthalten eingesetzt werden. Die eingesetzten Zellen können dabei nativ, permeabilisiert oder lysiert vorliegen.

Je kg umzusetzender Verbindung der Formel 1 können 10 000 bis bevorzugt 10 Mio Einheiten (U) Oxidoreduktase eingesetzt. Der Enzymeinheit 1 U entspricht dabei der Enzymmenge die benötigt wird, um 1 µmol der Verbindung der Formel Ije Minute (min) umzusetzen.

Dem Wasser kann ein Puffer zugesetzt werden, beispielsweise Kaliumphosphat-, Tris/HCI- oder Triethanolamin-Puffer mit einem pH-Wert von 5 bis 10, vorzugsweise einem ph-werft von 6 bis 9.

Der Puffer kann zusätzlich noch Ionen zur Stabilisierung des Enzyms enthalten, beispielsweise Magnesiumionen.

Desweiteren kann im erfindungsgemäßen Verfahren noch ein weiterer Stabilisator der Alkohol-Dehydrogenase eingesetzt werden, wie beispielsweise Glycerin, Sorbitol, 1,4 -DL-Dithiothreit (DTT) oder Dimethylsulfoxid (DMSO).

Die Konzentration des Cofaktors NAD(P)H bezogen auf die wässrige Phase beträgt von 0,001 mM bis 1 mM, insbesondere von 0,01 mM bis 0,1 mM.

Die Temperatur beträgt beispielsweise von etwa 10 °C bis 60 °C, bevorzugt von 20 °C bis 35 °C.

Eine Verfahrensvariante zur Erhöhung des Umsatzes der Ketoverbindung ist dabei die, dass das oxidierte Cosubstrat im Verfahren aus dem Reaktionsgemisch entweder schrittweise oder kontinuierlich entfernt wird.

Desweiteren kann dem Reaktionsansatz schrittweise oder kontinuierlich frisches Cosubstrat; Enzym oder Cofaktor zugeführt werden.

Das erfindungsgemäße Verfahren wird beispielsweise in einem Reaktionsgefäß aus Glas oder Metall durchgeführt. Dazu werden die Komponenten einzeln in das Reaktionsgefäß überführt und unter einer Atmosphäre von beispielsweise Stickstoff oder Luft gerührt. Je nach Substrat und eingesetzter Verbindung der Formel I beträgt die Reaktionszeit von 1 Stunde bis 96 Stunden, insbesondere von 2 Stunden bis 24 Stunden.

Mit den nachfolgenden Beispielen werden bevorzugte Ausführungsformen der Erfindung näher erläutert.

Die Reduktion der Verbindungen der Formel 1 wird zweckmäßigerweise so durchgeführt, daß die untenstehenden Komponenten in ein Reaktionsgefäß übergeführt und bei guter Durchmischung bei Raumtemperatur inkubiert werden. Nach Beendigung der Reaktion kann das Produkt je nach Löslichkeit durch Extraktion aus der wässrigen Reaktionslösung, durch Destillation aus der Reaktionslösung oder durch Kombination von Extraktion und Destillation isoliert und gereinigt werden.

In allen nachstehenden Beispielen wurden die Enzyme in Form von Rohextrakten eingesetzt.

### Beispiel 1: Synthese von (S)-Ethyl-4-chloro-3-hydroxybuttersäure

| Komponente | Menge | Prozentualer Anteil am Reaktionsvolumen | Konzentration |
|---|---|---|---|
| Puffer | | | |
| (TEA pH = 7, | 60 ml | | |
| 2mM MgCl₂,) | | | |
| NADP [M = 765 | 4,8 mg | | = 6,3 µmol |
| g/mol] | | | = 0,015 mM |
| Cosubstrat 2-Propanol | 200 ml | 50 % | |
| Ethyl -4- | 80 ml = 96 g | 20 % (v/v) | |
| chloracetoacetat | | 24 % (w/v) | 0,58 mol |
| Enzym | | | |
| = R-ADH aus L.minor | 60 000 Units | | |
| =1000 U/ml | (60 ml) | | |
| Volumen | 400 ml | | |
| Inkubationszeit | 24 h | | |
| Umsatz | >99 % | | |
| ee-Wert | >99,9 % S | | |
| ttn NADP | 92 950 | | |
| Enzymverbrauch | 600 000 Units/kg | | |

### Beispiel 2: Synthese von (R)-Methyl-3-hydroxybuttersäure

| Komponente | Menge | Prozentualer Anteil am Reaktionsvolumen | Konzentration |
|---|---|---|---|
| Puffer | | | |
| (TEA pH = 7,1mM MgCl₂, 10 % Glycerin) | 300 ml | | |
| NADP [M = 765 g/mol] | 10 mg | | 13 µmol (0,012 mM) |
| Cosubstrat | | | |
| 2-Propanol | 400 ml | 36,6 % | |
| Methylacetoacetat | 300 ml | 27,5 %(v/v) | |
| M=116 g/mol, d= 1,077 g/cm³ | | 29,6 (w/v) | 2,7 mol |
| Enzym | | | |
| = R-ADH aus L.minor | 90 000 Units | | |
| = 1000 U/ml | 90 ml | | |
| Volumen | 1090 ml | | |
| Inkubationszeit | 24 h | | |
| Umsatz | 99 % | | |
| ee-Wert | > 99,9 % | | |
| ttn NADP | 207692 | | |
| Enzymverbrauch | 280 000 U/kg | | |

### Beispiel 3: Synthese von Ethyl-D-lactat

| Komponente | Menge | Prozentualer Anteil am Reaktionsvolumen | Konzentration |
|---|---|---|---|
| Puffer | | | |
| (TEA pH = 7,1 mM MgCl₂, | 170 ml | | |
| 10 % Glycerin) | | | |
| NADP [M = 765 g/mol] | 40 mg | | 52 µmol (0,054mM) |
| Cosubstrat | | | |
| 2-Propanol | 500 ml | 52 % | |
| | | | |
| Ethyl-pyruvat | 250 ml | 26 % (v/v) | 2,2 mol |
| M = 117 g/mol, d = 1.045) | | 27,2 (w/v) | |
| Enzym | | | |
| = R-ADH aus L.minor | 40 000 | | |
| = 1000 U/ml | | | |
| Volumen | 960 ml | | |
| Inkubationszeit | 48 h | | |
| Umsatz | 99 % | | |
| ee-Wert | l> 99 % | | |
| ttn NADP | 42 300 | | |
| Enzymverbrauch | 160 000 U/kg | | |

### Beispiel 4: Synthese von (R)-1,3 Butandiol

| Komponente | Menge | Prozentualer Anteil am Reaktionsvolumen | Konzentration |
|---|---|---|---|
| Puffer (TEA pH = 7,1mM MgCl_{2,} 10 % Glycerin) | 0,5 ml | | |
| NADP [M = 765 g/mol] | 0,1 mg | | 0,13 µmol (0,013 mM) |
| Cosubstrat | 4,5 ml | | |
| 2-Propanol | | 44% | |
| 4-Hydroxy-2-butanon | 5 ml | | |
| (M =88,12 gmol) | | 48 % (v/v) | 0,057 mol |
| Enzym (R-ADH aus | 250 U | | |
| L.minor) | (250 µl) | | |
| = 1000 U/ml | | | |
| Volumen | 10,25 ml | | |
| System: | Monophasisch | | |
| Prozessführung*: | Abdestillieren des Aceton | | |
| | Zugabe 2-Propanol stufenweise | | |
| Inkubationszeit | 24 h | | |
| Gesamtverbrauch 2-Propanol | 13,5 ml | | |
| Umsatz | 90 % | | |
| ee-Wert | 99 % R | | |
| ttn NADP | 438461 | | |
| Enzymverbrauch | 750 000 U/kg | | |

| | | | |
|---|---|---|---|
| *Aus dem Ansatz wurde zweimal das entstandene Aceton abdestilliert und anschließend dem Reaktionsgemisch erneut die gleiche Menge 2-Propanol und Enzym zugegeben, wie zu Beginn der Reaktion. So konnte selbst in einem Ansatz mit 48 % Substratkonzentration 90 % Umsatz erreicht werden. | | | |

### Beispiel 5: Synthese von R-2-Oktanol

| Komponente | Menge | Prozentualer Anteil am Reaktionsvolumen | Konzentration |
|---|---|---|---|
| Puffer (TEA pH = 7,1 mM | | | |
| MgCl₂, 10 % Glycerin) | 270 ml | | |
| NADP [M = 765 g/mol] | 27 mg | | 35 µmol |
| | | | (=0,023 mM) |
| Cosubstrat | | | |
| 2-Propanol | 900 ml | 60 % | |
| 2-Oktanon | | | |
| (128 g/mol, d= 0,8) | 300 ml | 20 % (v/v) | 1,87 M |
| | | 16 % (w/v) | |
| Enzym (R-ADH aus | | | |
| L.minor) | 30 000 Units | | |
| = 1000 U/ml | (30 ml) | | |
| Volumen | 1500 | | |
| System: | monophasisch | | |
| Prozessführung*: | Abdestillieren des Aceton | | |
| | Zugabe 2-Propanol stufenweise | | |
| Inkubationszeit | 24 h | | |
| Gesamtverbrauch 2-Propanol | 1350 ml | | |
| Umsatz | 97 % | | |
| ee-Wert | 100 % R | | |
| ttn NADP | 53 000 | | |
| Enzymverbrauch | 200 000 U/kg | | |

| | | | |
|---|---|---|---|
| *Aus dem Ansatz wurde einmal das entstandene Aceton abdestilliert und anschließend dem Reaktionsgemisch erneut die gleiche Menge 2-Propanol und Enzym zugegeben, wie zu Beginn der Reaktion. So konnte selbst in einem Ansatz mit 20 % Substratkonzentration 97 % Umsatz erreicht werden. | | | |

### Beispiel 6: Synthese von (R,R)-2,5-Hexandiol

| Komponente | Menge | Prozentualer Anteil am Reaktionsvolumen | Konzentration |
|---|---|---|---|
| Puffer (TEA pH = 6,1 mM | | | |
| MgCl₂, 10 % Glycerin) | 100 ml | | |
| NADP [M = 765 g/mol] | 5 mg | | 6,5 µmol (0,011mM) |
| Cosubstrat 2-Propanol | 325 ml | 56 % | |
| 2,5 Hexandion | 125 ml | 22 % (v/v) | 1,09 mol |
| (114 g/mol, d= 1) | | 22 % (w/v) | |
| Enzym (R-ADH aus L.minor)= 1000 U/ml | 25 000 | | |
| Volumen | 575 ml | | |
| System: | monophasisch | | |
| Prozessführung*: | Abdestillieren des Aceton | | |
| | Zugabe 2-Propanol stufenweise | | |
| Inkubationszeit | 48 h | | |
| Gesamtverbrauch 2-Propanol | 650 ml | | |
| Umsatz | 78 % | | |
| ee-Wert | 100 % R,R | | |
| ttn NADP | 168 000 | | |
| Enzymverbrauch | 400 000 U/kg | | |

Aus dem Ansatz wurde einmal das entstandene Aceton abdestilliert und anschließend dem Reaktionsgemisch erneut die gleiche Menge 2-Propanol und Enzym zugegeben, wie zu Beginn der Reaktion.

### Beispiel 7: Synthese von (S)-Ethyl-4-chloro-3-hydroxybuttersäure

| Komponente | Menge | Prozentualer Anteil am Reaktionsvolumen | Konzentration |
|---|---|---|---|
| Puffer (TEA pH = 7, 2mM MgCl₂,) | 2 ml | | |
| NADP [M = 765 g/mol] | 2 mg | | = 2,6 µmol |
| | | | = 0,065 mM |
| Cosubstrat | | | |
| 2-Propanol | 30 ml | 65 % | |
| Ethyl -4-chloracetoacetat | 8 ml = 9,6 g | 17 % (v/v) | |
| | | 20 % (w/v) | 58 mmol |
| Enzym | | | |
| = R-ADH aus Leuconostoc | 67 00 Units | | |
| camosum DSMZ 5576 = 1000 U/ml | (6 ml) | | |
| Volumen | 46 ml | | |
| Inlcubationszeit | 24 h | | |
| Umsatz | >99 % | | |
| ee-Wert | >99,9 % S | | |
| ttn NADP | 22 300 | | |
| Enzymverbrauch | 670 000 Units/kg | | |

### Beispiel 8: Synthese von (1R)-1- [3,5-bis(täfluoromethyl)phenyl] lethan-1-ol

| Komponente | Menge | Prozentualer Anteil am Reaktionsvolumen | Konzentration |
|---|---|---|---|
| Puffer | | | |
| (TEA pH = 8,5, 2mM MgCl₂,) | 200 µl | | |
| NAD [M = 663 g/mol] | 0,05 mg | | 0,075 µmol |
| | | | (0,027 mM) |
| Cosubstrat 2-Propanol | 250 µl | 41,6 % (v/v) | |
| 1 - [3,5 bis-(trifluoro-methyl)phenyl] ethan-1-on [256.15 g/mol] d=1,422 | 100 µl | 16,6 % (v/v) | |
| Enzym | 40 Units | | 0,56 mmol |
| = R-ADH aus Pichia finlandica (EP1179595A1) | (0,05 ml) | | |
| Volumen | 600 µl | | |
| Inkubationszeit | 24 h | | |
| Umsatz | 99 % | | |
| ee-Wert | 99,9 % R | | |
| ttn NAD | ca. 7500 | | |
| Enzymverbrauch | 285 000 U/kg | | |

## Patentansprüche

1. Verfahren zur enantioselektiven enzymatischen Reduktion von Ketoverbindungen der allgemeinen Formel I
R₁-C(O)-R₂ (I)
in der R1 für einen der Reste
1) -(C₁-C₂₀)-Alkyl, worin Alkyl geradkettig oder verzweigtkettig ist,
2) -(C₂-C₂₀)-Alkenyl, worin Alkenyl geradkettig oder verzweigtkettig ist und gegebenenfalls bis zu vier Doppelbindungen enthält,
3) -(C₂-C₂₀)-Alkinyl, worin Alkinyl geradkettig oder verzweigtkettig ist und gegebenenfalls bis zu vier Dreifachbindttngen enthält,
4) -(C₆-C₁₄)-Aryl,
5) -(C₁-C₈)-Alkyl-(C₆-C₁₄)-Aryl ,
6) -(C₅-C₁₄₎-Heterocyclus, der unsubstituiert oder ein-, zwei- oder dreifach durch -OH, Halogen, -NO₂ und/oder -NH₂ substituiert ist, oder
7) -(C₃-C₇)-Cycloalkyl;
steht, wobei die oben unter 1) bis 7) genannten Reste unsubstituiert sind oder unabhängig voneinander ein-, zwei- oder dreifach durch -OH, Halogen, -NO₂ und/oder -NH₂ substituiert sind,
und R₂ für einen der Reste
8) -(C₁-C₆)-Alkyl, worin Alkyl geradkettig oder verzweigtkettig ist,
9) -(C₂-C₆)-Alkenyl, worin Alkenyl geradkettig oder verzweigtkettig ist und gegebenenfalls bis zu drei Doppelbindungen enthält,
10) -(C₂-C₆)-Alkinyl, worin Alkinyl geradkettig oder verzweigtkettig ist und gegebenenfalls zwei Dreifachbindungen enthält, oder
11) -(C₁-C₁₀)-Alkyl--C(O)-O-(C₁-C₆)-Alkyl, worin Alkyl gerade oder verzweigtkettig ist und unsubstituiert ist oder ein-, zwei- oder dreifach durch -OH, Halogen, -NO₂ und/oder - NH₂ substituiert ist,
steht, wobei die oben unter 8) bis 11) genannten Reste unsubstituiert sind oder unabhängig voneinander ein-, zwei- oder dreifach durch -OH, Halogen, -NO₂ und/oder
-NH₂ substituiert sind,
**dadurch gekennzeichnet, daß**
eine flüssige, einphasige Mischung, welche
(a) mindestens 5 Gew./Vol.-% eine Verbindung der Formel (I),
(b) mindestens 15 Vol.-% 2-Propanol und/oder 2-butanol, und
(c) Wasser
enthält, mit einer R-spezifischen Oxidoreduktase in Gegenwart eines Co-Faktors behandelt wird, um eine chirale Hydroxyverbindung der allgemeinen Formel II
R₁-CH(OH)-R₂ (II)
zu bilden, in welcher R₁ und R₂ die oben angegebene Bedeutung haben,
wobei der Co-Faktor durch Oxidation des 2-Propanols und/oder 2-Butanols regeneriert wird.

2. Verfahren zur enantioselektiven enzymatischen Reduktion der Ketoverbindung 2,5-Hexandion, **dadurch gekennzeichnet, daß** eine flüssige, einphasige Mischung, welche
(a) mindestens 5 Gew./Vol.-% 2,5-Hexandion,
(b) mindestens 15 Vol.-% 2-Propanol und/oder 2-Butanol, und
(c) Wasser
enthält, mit einer R-spezifischen Oxidoreduktase in Gegenwart eines Co-Fah-tors behandelt wird, wobei der Co-Faktor durch Oxidation des 2-Propanols und/oder 2-Butanols regeneriert wird.

3. Verfahren zur enantioselektiven enzymatischen Reduktion der Ketoverbindung Acetoxyaceton, **dadurch gekennzeichnet, daß** eine flüssige, einphasige Mischung, welche
(a) mindestens 5 Gew./Vol.-% Acetoxyaceton,
(b) mindestens 15 Vol.-% 2-Propanol und/oder 2-Butanol, und
(c) Wasser
enthält, mit einer R-spezifischen Oxidoreduktase in Gegenwart eines Co-Faktors behandelt wird, wobei der Co-Faktor durch Oxidation des 2-Propanols und/oder 2-Butanols regeneriert wird.

4. Verfahren nach einem der Ansprüche 1 bis 3, **dadurch gekennzeichnet, daß** die R-spezifische Oxidoreduktase mikrobiellen Ursprungs ist und insbesondere aus Bakterien der Gruppe Lactobacillales, insbesondere der Gattung *Lactobacillus,* oder aus Hefen, insbesondere der Gattungen *Pichia, Candida, Pachysolen, Debaromyces* oder *Issatschenkia* stammt.

5. Verfahren nach einem der Ansprüche 1 bis 4, **dadurch gekennzeichnet, daß** als Co-Faktor NAD(P)H eingesetzt wird.

6. Verfahren nach einem der Ansprüche 1 bis 5, **dadurch gekennzeichnet, daß** die flüssige, einphasige Mischung bei Anwendung einer Oxidoreduktase bakteriellen Ursprungs mindestens 25 Vol.-% 2-Propanol und/oder 2-Butanol enthält.

7. Verfahren nach einem der Ansprüche 1 bis 5, **dadurch gekennzeichnet, daß** die flüssige, einphasige Mischung zwischen 25 und 90 Vol.-%, insbesondere zwischen 35 und 70 Vol.-%, 2-Propanol und/oder 2-Butanol enthält.

8. Verfahren nach einem der Ansprüche 1 bis 7, **dadurch gekennzeichnet, daß** die flüssige, einphasige Mischung die Ketoverbindung zwischen 5 und 50 Gew./Vol.%, insbesondere zwischen 15 und 50 Gew./Vol.%, enthält.

9. Verfahren nach einem der Ansprüche 1 und 4 bis 8, **dadurch gekennzeichnet, daß** als Verbindung der allgemeinen Formel (I) Ethyl-4-chloracetoacetat, Methylacetoacetat, Ethyl-3-oxovaleriat, 4-Hydroxy-2-butanon, Ethylpyruvat, 2,3-Dichloracetophenon, Acetophenon, 1-[3,5-Bis(trifluoromethyl)pheny]ethan-1-on, 2-Oetanon, 3-Octanon , 1,4-Dichlor-2-butanon, Phenacylchlorid, Ethyl-4-bromoacetoacetat, 1,1-Dichloraceton, 1,1,3-Trichloraceton oder 1-Chloraceton eingesetzt wird.

## Claims

1. A process for the enantioselective enzymatic reduction of keto compounds of general formula I
R₁-C(O)-R₂ (I)
in which R1 stands for one of the moieties
1) -(C₁-C₂₀)-alkyl, wherein alkyl is linear-chain or branched,
2) -(C₂-C₂₀)-alkenyl, wherein alkenyl is linear-chain or branched and optionally contains up to four double bonds,
3) -(C₂-C20)-alkynyl, wherein alkynyl is linear-chain or branched and optionally contains up to four triple bonds,
4) -(C₆-C₁₄)-aryl,
5) -(C₁-C₈)-alkyl-(C₆-C₁₄)-aryl,
6) -(C₅-C₁₄)-heterocycle which is unsubstituted or substituted one, two or three times by -OH, halogen, -NO₂ and/or -NH₂, or
7) -(C₃-C₇)-cycloalkyl,
wherein the moieties mentioned above under 1) to 7) are unsubstituted or substituted one, two or three times, independently of each other, by -OH, halogen, -NO₂ and/or -NH₂,
and R₂ stands for one of the moieties
8) -(C₁-C₆)-alkyl, wherein alkyl is linear-chain or branched,
9) -(C₂-C₆)-alkenyl, wherein alkenyl is linear-chain or branched and optionally contains up to three double bonds,
10) -(C₂-C₆)-alkynyl, wherein alkynyl is linear-chain or branched and optionally contains two triple bonds, or
11) -(C₁-C₁₀)-alkyl-C(O)-O-(C₁-C₆)-alkyl, wherein alkyl is linear or branched and is unsubstituted or substituted one, two or three times by -OH, halogen, -NO₂ and/or -NH₂, wherein the moieties mentioned above under 8) to 11) are unsubstituted or substituted one, two or three times, independently of each other, by -OH, halogen, -NO₂ and/or NH₂,
**characterized in that**
a liquid, single-phase mixture comprising
(a) at least 5% by weight/by volume of a compound of formula (I),
(b) at least 15% by volume of 2-propanol and/or 2-butanol, and
(c) water
is treated with an R-specific oxidoreductase in the presence of a cofactor in order to form a chiral hydroxy compound of general formula II
R₁-CH(OH)-R₂ (II)
wherein R₁ and R₂ have the above-indicated meaning,
whereby the cofactor is regenerated by oxidation of the 2-propanol aud/or the 2-butanol.

2. A process for the enantioselective enzymatic reduction of the keto compound 2,5-hexanedione, **characterized in that** a liquid, single-phase mixture comprising
(a) at least 5% by weight/by volume of 2,5-hexanedione,
(b) at least 15% by volume of 2-propanol and/or 2-butanol, and
(c) water
is treated with an R-specific oxidoreductase in the presence of a cofactor, whereby the cofactor is regenerated by oxidation of the 2-propanol and/or the 2-butanol.

3. A process for the enantioselective enzymatic reduction of the keto compound acetoxyacetone, **characterized in that** a liquid, single-phase mixture comprising
(a) at least 5% by weight/by volume of acetoxyacetone,
(b) at least 15% by volume of 2-propanol and/or 2-butanol, and
(c) water
is treated with an R-specific oxidoreductase in the presence of a cofactor, whereby the cofactor is regenerated by oxidation of the 2-propanol and/or the 2-butanol.

4. A process according to any of claims 1 to 3, **characterized in that** the R-specific oxidoreductase is of microbial origin and stems in particular from bacteria of the group of Lactobacillales, particularly of the genus *Lactobacillus,* or from yeasts, particularly of the genera *Pichia, Candida, Pachysolen, Debaromyces* or *Issatschenkia.*

5. A process according to any of claims 1 to 4, **characterized in that** NAD(P)H is used as the cofactor.

6. A process according to any of claims I to 5, **characterized in that** the liquid, single-phase mixture contains at least 25% by volume of 2-propanol and/or 2-butanol if an oxidoreductase of a bacterial origin is used.

7. A process according to any of claims 1 to 5, **characterized in that** the liquid, single-phase mixture contains between 25 and 90% by volume, in particular between 35 and 70% by volume, of 2-propanol and/or 2-butanol.

8. A process according to any of claims 1 to 7, **characterized in that** the liquid, single-phase mixture contains the keto compound in an amount of between 5 and 50% by weight/by volume, in particular of between 15 and 50% by weight/by volume.

9. A process according to any of claims 1 and 4 to 8, **characterized in that** ethyl-4-chloroacetoacetate, methylacetoacetate, ethyl-3-oxovaleriate, 4-hydroxy-2-butanone, ethylpyruvate, 2,3-dichloroacetoplienone, acetophenone, 1-[3,5-bis(trifiluoromethyl)-pheuyl]ethane-1-one, 2-octanone, 3-octanone, 1,4-dichloro-2-butanone, phenacylchloride, ethyl-4-bromoacetoacetate, 1,1-dichloroacetone, 1,1,3-trichloroacetone or 1-chloroacetone is used as the compound of general formula (I).

## Revendications

1. Procédé de réduction enzymatique énantiosélective de composés cétoniques de formule générale 1
R₁-C(O)-R₂ (I)
dans laquelle R₁ représente un des groupes
1) alkyle en C₁ à C₂₀, l'alkyle étant à chaîne linéaire ou à chaîne ramifiée,
2) alcényle en (C₂ à C₂₀), l'alcényle étant à chaîne linéaire ou à chaîne ramifiée et contenant éventuellement jusque quatre doubles liaisons,
3) alcinyle en C₂ à C₂₀, l'alcinyle étant à chaîne linéaire ou à chaîne ramifiée et contenant éventuellement jusque quatre triples liaisons,
4) aryle en C₆ à C₁₄,
5) (alkyle en C₁ à C₈)(aryle en C₆ à C₁₉),
6) hétérocycle en C₅ à C₁₄, non substitué ou substitué une, deux ou trois fois par -OH, un halogène, - NO₂ et/ou -NH₂ ou
7) cycloalkyle en C₃ à C₇,
les groupes cités ci-dessus en 1) à 7) n'étant pas substitués ou étant substitués indépendamment les uns des autres une, deux ou trois fois par -OH, un halogène, -NO₂ et/ou -NH₂,
et R₂ représentant l'un des groupes :
8) alkyle en C₁ à C₆, l'alkyle étant à chaîne linéaire ou à chaîne ramifiée,
9) alcényle en C₂ à C₆, l'alcényle étant à chaîne linéaire ou à chaîne ramifiée et contenant éventuellement jusque trois doubles liaisons,
10) alcinyle en C₂ à C₆, l'alcinyle étant à chaîne linéaire ou à chaîne ramifiée et contenant éventuellement deux triples liaisons ou
11) (alkyle en C₁ à C₁₀)-C(O)-O- (alkyle en C₁ à C₆), l'alkyle étant à chaîne linéaire ou à chaîne ramifiée et étant non substitué ou substitué une, deux ou trois fois avec -OH, un halogène, -NO₂ et/ou -NH₂,
les groupes cités ci-dessus en 8) à 11) étant non substitués ou substitués indépendamment les uns des autres deux ou trois fois par -OH, un halogène, -NO₂ et/ou -NH₂,
**caractérisé en ce que**
un mélange monophasique liquide qui contient
(a) au moins 5 % en poids ou en volume d'un composé de formule (I),
(b) au moins 15 % en volume de 2-propanol et/ou de 2-butanol et
(c) de l'eau,
est traité avec une oxydase R-spécifique en présence d'un co-facteur pour former un composé hydroxylé chiral de formule générale II
R₁-CH (OB) -R₂ (II)
dans laquelle R₁ et R₂ ont les significations données plus haut, le co-facteur étant régénéré par oxydation du 2-propanol et/ou du 2-butanol.

2. Procédé de réduction enzymatique énantiosélective du composé cétonique 2,5-hexanedione, **caractérisé en ce qu'**un mélange monophasiqus qui contient
(a) au moins 5 % en poids ou en volume de 2,5-hexanedione,
(b) au moins 15 % en volume de 2-propanol et/ou de 2-butanol et
(c) de l'eau,
est traité avec une oxydase R-spécifique en présence d'un co-facteur, le co-facteur étant régénéré par oxydation du 2-propanol et/ou du 2-butanol.

3. Procédé de réduction enzymatique énantiosélective du composé cétonique acétoxyacétone, **caractérisé en ce qu'**un mélange monophasique liquide qui contient
(a) au moins 5 % en poids ou en volume d'acétoxyacétone,
(b) au moins 15 % en volume de 2-propanol et/ou de 2-butanol et
(c) de l'eau,
est traité avec une oxydase R-spécifique en présence d'un co-facteur acétoxyacétone, le co-facteur étant régénéré par oxydation du 2-propanol et/ou du 2-butanol.

4. Procédé selon l'une des revendications 1 à 3, **caractérisé en ce que** l'oxydoréductase R-spécifique est d'origine microbienne et provient en particulier de bactéries du groupe Lactobacillales, en particulier de l'espère *Laccobacillus,* ou de levures, en particulier des espèces Pichia, *Candida, Pachysoleri,* Debaxomyces ou *Issatschenkia.*

5. Procédé selon l'une des revendications 1 à 4, **caractérisé en ce qu'**il utilise comme co-facteur le NAD(P)H.

6. Procédé selon l'une des revendications 1 à 5, **caractérisé en ce que** le mélange monophasique liquide contient au moins 25 % en volume de 2-propanol et/ou de 2-butanol lorsqu'il est utilisé avec une oxydoréductase d'origine bactérienne.

7. Procédé selon l'une des revendications 1 à 5, **caractérisé en ce que** le mélange monophasique liquide contient entre 25 et 90 % en volume et en particulier entre 35 et 70 % en volume de 2-propanol et/ou de 2-butanol.

8. Procédé selon l'une des revendications 1 à 7, **caractérisé en ce que** dans le mélange monophasique liquide, composé cétonique a une teneur comprise entre 5 et 50 % en volume et en particulier entre 15 et 50 % en volume.

9. Procédé selon l'une des revendications 1 et 4 à 8, **caractérisé en ce que** le composé de formule générale (I), il utilise le 4-chloroacétoacétate d'éthyle, l'acétoacétate de méthyle, le 3-oxovalérate d'éthyle, la 4-hydroxy-2-butanone, le pyruvate d'éthyle, la 2,3-dichloroacétophénone, l'acétophénone, la 1-[3,5-bis(trifluorométhyl)phénylléthane-1-one, la 2-octanone, la 3-octanone, à 1,4-dichloro-2-butanone, le chlorure de phénacyle, le 4-bromoacétoacétate d'éthyle, la 1,1-dichloroacétone, la 1,1,3-trichloroacétone ou la 1-chloroacétone.
